# NEW EUROPEAN PATENT SPECIFICATION

(11) **EP 0 243 069 B2**
(45) Date of publication and mention of the opposition decision: **09.06.1999**
(45) Mention of the grant of the patent: 11.11.1992
(21) Application number: 87303252.8
(22) Date of filing: 14.04.1987
(51) Int. Cl.: A61L 15/16, A61F 13/02

(54) **Method of making wound dressings**
Methode zur Herstellung von Wundpflaster
Méthode pour la préparation des pansements pour blessure

(30) Priority: 17.04.1986 GB 8609367
(43) Date of publication of application: 28.10.1987
(73) Proprietor: JOHNSON & JOHNSON MEDICAL, INC., Arlington, Texas 76014 (US)
(72) Inventor: Wiggans, Richard John, Ingleton Carnforth Lancashire (GB); Squires, Leslie James, Shedfield Wickham Hampshire (GB); Smith, Rory James Maxwell, Near Grassington North Yorkshire (GB)
(74) Representative: Fisher, Adrian John

(56) References cited:
- EP-A- 0 072 680
- EP-A- 0 099 758
- EP-A- 0 117 632
- EP-A- 0 123 465
- EP-A- 0 140 596
- EP-A- 0 236 104
- GB-A- 976 301
- GB-A- 1 255 155
- GB-A- 1 280 631
- GB-A- 1 328 088
- GB-A- 1 329 693
- GB-A- 1 379 158
- GB-A- 2 131 701
- US-A- 4 393 048
- "Advances in Wound Management", The Surgical Dressings Research Unit, Welsh School of Pharmacy, Univ. of Wales Inst. of Sc. and Techn, Cardiff, GB, 1986, pp. 53-81
- A. R. Groves et al, "Alginate dressing as a donor site haemostat", Annals of the Royal College of Surgeons of England, 1986, vol 68
- R. Fraser et al, "Sorbsan calcium alginate fibre dressings in footcare", Biomaterials, 1983, vol. 4, pp. 222-224

## Description

This invention relates to methods of making wound dressings.

One known type of wound dressing comprises a thin plastics film which has an adhesive coating on one surface. This group of dressings includes the so-called occlusive dressings, by which is meant dressings which maintain the wound in a relatively moist state in which tissue repair is found to be enhanced. One form of occlusive dressing is that described in EP-A-0117632, and sold under the Trade Mark BIOCLUSIVE by Johnson & Johnson. It consists of a thin polyurethane film (of the order of 35 to 44 micron thickness), coated on one surface with an acrylic adhesive.

An alternative, and well-known, form of dressing comprises an absorbent pad attached to an adhesive backing, which backing is often liquid permeable and, even when not liquid permeable, is usually highly permeable to moisture vapour. Such dressings are very commonly used for minor injuries and also in more serious cases when the volume of wound exudate is particularly large.

The present invention concerns a method of making wound dressings which combine the rapid healing advantages of occlusive dressings with the satisfactory management of wound exudate. These dressings comprise an adhesive-coated plastics film to which is attached a layer of absorbent material, such that a region of the adhesive-coated plastics film surrounds the absorbent material.

One difficulty associated with the attachment of layers of absorbent material having a low degree of coherence, such as powders or fibres, is that particles of the material tend to be shed and adhere to adjoining regions of the adhesive, where they impair adhesion of the dressing to the patient's skin. The present invention provides an economical method for avoiding this difficulty.

According to the present invention, there is provided a method of forming a wound dressing comprising the steps of
i) providing an adhesive-coated plastics film having a lamella releasably attached to its adhesive surface,
ii) cutting the lamella into an inner and an outer portion, such that the outer portion surrounds the inner portion,
iii) removing the inner portion to expose a region of the adhesive-coated plastics film, and
iv) attaching a layer of absorbent material to said exposed region of film.

In a particularly preferred embodiment, the outer portion of lamella is itself then removed in whole or in part to expose a further region of adhesive-coated film, to which may be attached one or more protective films to prevent soiling of the absorbent layer prior to use.

Any part of the outer portion of lamella which is left in place for removal at the time of use of the dressing may be provided with a handling tab for ease of such removal. Similarly, if in the manufacture of the dressing a region of adhesive coated film is exposed and subsequently protected with a release lamella, such release lamella is preferably also provided with a handling tab. The handling tab or tabs may be integral with the lamella, as in the BIOCLUSIVE dressing, but it is preferred that it or they be adhesively secured to the lamella, as disclosed in EP-A-0120570.

A handling tab may also be secured to the inner portion of the lamella and to any part of the outer portion which is removed in the manufacturing process. Such handling tab or tabs will of course not be used by the end user of the dressing, but as an aid to manufacture.

If it is desired to attach handling tabs to two adjacent portions of lamella, the following method is preferred. Firstly, a strip of handling tab material is attached to the lamella, bridging the intended line of cut separating the two portions of lamella. The handling tab material and the lamella are then cut simultaneously to provide a handling tab on each side of the line of cut.

The release lamella may be formed from any of a wide variety of materials, but it will usually be formed from paper, from a plastics film, or from a combination of paper and plastics film. In order to provide ready release from the adhesive which will be used to adhere the dressing to the patient, the lamella, if formed from paper, will usually be coated with a silicone polymer in conventional manner. However, it is preferred that the outer-facing surface not be silicone-coated, so that it is receptive to adhesive for securing handling tabs. In practice, it is difficult to coat relatively lightweight paper with silicone on one surface only (because paper is somewhat permeable to the silicone), and it is therefore preferred to use a paper/plastics laminate as the material of the lamella. Such paper/plastics laminates are substantially impermeable to silicone and can therefore readily be silicone-coated on only one surface. The non-coated surface, in addition to being receptive to the adhesive for the handling tabs, is also receptive to printing inks. The release lamellae of the dressings of the present invention can therefore be printed as desired.

The handling tabs may be of the same material as the release lamella, or of different material. They may, for example, be of fabric or of uncoated paper. These materials have the advantage of giving better grip than the handling tabs of BIOCLUSIVE dressings which are, of necessity, silicone-coated.

The adhesive which is used for securing handling tabs to the release lamella is preferably a hot-melt adhesive such as a ethylene/vinyl acetate copolymer. One example of a suitable adhesive is that available under the Trade Mark INSTANT LOK from National Adhesives & Resins Ltd. The adhesive is conveniently extruded onto the lamella in a strip from 1 mm to 5 mm wide, and from 0.5 g/m to 3 g/m. It will be appreciated, of course, that the adhesive can alternatively be applied to the handling tabs.

The lamella can be cut into the desired separate portions, without cutting the plastics film, by techniques already known in the adhesive labels art, such as by die-stamping. As is well known in that art, it is more straightforward to cut through a soft material against a hard one than vice versa. However, the precision of modern die-cutting machinery (such as that made by Edale Engineering Ltd. of Budds Lane, Romsey, Hampshire, England) is such as to allow even a relatively hard release lamella to be cut without damaging the underlying, relatively soft, plastics film. The critical requirement is that the release lamella be of closely defined thickness, so that the machine can be adjusted to cause the knives to penetrate just the thickness of the lamella and no further.

The ability of alginates to form haemostatic gels on coming into contact with body fluids is already known, but we have found that this property is of particular advantage in occlusive dressings. In some cases, fluid accumulation under an occlusive dressing can be troublesome, but the incorporation of an alginate layer in an occlusive dressing means that any fluid exuded by the wound is rapidly immobilised by the alginate.

The alginate may, for example, be calcium alginate, silver alginate, zinc alginate, sodium alginate, or mixtures thereof. Calcium alginate is particularly preferred, and is available from Courtaulds PLC, England.

The alginate layer may conveniently be in the form of a pad of alginate fibres, such as fibres having a count in the range 1 decitex to 3.3 decitex. The preferred fibre length is from 10 to 100 mm, with a fibre length of from 25 to 75 mm being particularly preferred.

When a fibrous alginate pad is used, it will usually be lightly bonded together to give a degree of integrity and resistance to delamination. This stabilisation can be achieved by any of a variety of means such as calendering, embossing or needling.

The thickness of the pad will generally be in the range 0.1 mm to 6 mm, and preferably from 0.5 mm to 3 mm. It is particularly preferred that the pad be from 1 to 2 mm in thickness. The weight per unit area of the pad will usually be from 30 g/m² to 300 g/m², and preferably from 70 g/m² to 150 g/m².

As an alternative to alginate fibres, alginates in powder form may be used. The thickness of the powder layer will generally be from 0.1mm to 0.75mm, and preferably from 0.05mm to 0.5mm. An average particle diameter of from 25 microns to 800 microns is preferred, with an average particle diameter in the range 50 microns to 400 microns being especially preferred. Although other alginates may be used if desired, the preferred powdered alginates are sodium alginate, calcium alginate, and mixtures thereof in any proportion. Suitable powdered alginates are available commercially from Kelco International, England.

A further form of alginate which is suitable for use in the dressings of the present invention is an alginate film. Such a film may be formed by casting a solution of a water soluble alginate, e.g. sodium alginate, followed by drying. Optionally, additional absorbent materials such as sodium carboxymethyl cellulose may be included, e.g. in an amount of from 2% to 25% by weight. A preservative agent such as 2-bromo-2-nitro-1,3-propanediol may also be added, as may a plasticiser such as glycerol. The preservative, if included, will generally be present in an amount of from 0.1% to 1% by weight, while a suitable amount of plasticiser is from 2% to 25% by weight. Thus, a typical formulation, after drying, might be:

| | |
|---|---|
| Sodium alginate | 79.5% to 89.8% w/w |
| Sodium carboxymethyl cellulose | 5% to 15% w/w |
| Preservative | 0.2% to 0.5% w/w |
| Glycerol | 5% to 15% w/w |

The dried film thickness will generally be from 0.01 mm to 1.0 mm, and preferably from 0.05 mm to 0.25 mm.

In a still further embodiment, the alginate may be in the form of a gel, generally from 0.01 to 2.0 mm in thickness. Such gels may suitably be formed by the controlled introduction of a suitable cation (e.g. calcium) into a solution of a water soluble alginate such as sodium alginate, preferably in the presence of a pH modifier such as glucono delta lactone. The alginate concentration may be, for example, from 2% to 30% by weight and the final cation concentration may suitably be from 0.2 to 10% by weight. Additional absorbents, plasticisers and preservatives may optionally be included, as described above in relation to alginate films. The resultant gel will typically contain from 30% to 99% by weight of water, and it may then be partially dried if desired, e.g. to a water content of from 15% to 50%, and more preferably from 20% to 40% by weight. A typical formulation might be

| | |
|---|---|
| sodium alginate | 5% to 15% w/w |
| sodium carboxymethyl cellulose | 5% to 15% w/w |
| Preservative | 0.2% to 0.5% w/w |
| Calcium phosphate | 0.5% to 4% w/w |
| Glycerol | 5% to 15% w/w |
| Glucono-δ-lactone | 0.5% to 4% w/w |
| Water to 100% | |

Whichever form of alginate is chosen, it may optionally contain an antimicrobial agent, and it may also contain other haemostatic agents. In the case of proteinaceous haemostatic agents, such as collagen and thrombin, the alginate may be in the form of a complex with the haemostatic protein. Suitable protein/alginate complexes are disclosed in EP-A-0140596.

The plastics film used in the dressings of the present invention will usually be substantially impervious to liquids. The film may, for example, be a continuous film of a non-adherent water-impermeable polymer such as a polyolefin. Polyethylene and polypropylene are representative examples of this class, but polymers of higher olefins may of course be used, as may copolymers of two or more olefins, or copolymers of an olefin and one or more other monomers. A suitable polyethylene film for use in the dressings of the present invention is available from Union Carbide Corporation under the Trade Mark UCAR, and a suitable polypropylene is available from British Cellophane Limited under the Trade Mark SHORCO.

Preferably, the film is impervious to liquids, but has a moisture vapour permeability in the range 1000 g/m²/24hr to 3000 g/m²/24hr. Continuous polyurethane films having such properties are available under the Trade Mark PLATILON from Plate Bonn GmbH., Bonn, W. Germany.

The film is preferably from 10 to 200 micrometers in thickness, and more preferably from 20 to 100 micrometers thick. For example, the film may be from 30 to 70 micrometers thick.

The adhesive which is used for adhering the dressing to the patient is preferably an acrylic adhesive such as a copolymer of bury acrylate and butyl methacrylate. It will usually be applied in an amount of from 20 g/m² to 50 g/m², and preferably from 35 g/m² to 45 g/m².

The size of the alginate pad will generally be related to the size of the dressing as a whole, but wide variations are possible. However, it is important that an adhesive coated margin of film surrounds the pad. This margin is preferably at least 10 mm wide, and more preferably at least 20 mm wide.

The dressings will usually be packaged in a hermetically-sealed envelope and sterilised, e.g with ethylene oxide or by γ-irradiation.

A wound dressing and a method of making it, are now described by way of example, with reference to the accompanying drawings, in which:-
Figure 1 is a schematic representation of a first stage in the manufacture of a dressing,
Figure 2 is a schematic representation of a second stage in the manufacture of a dressing,
Figure 3 is a schematic representation of a third stage in the manufacture of a dressing,
Figure 4 is a schematic representation of a fourth stage in the manufacture of a dressing, and
Figure 5 is a schematic representation of the completed dressing.

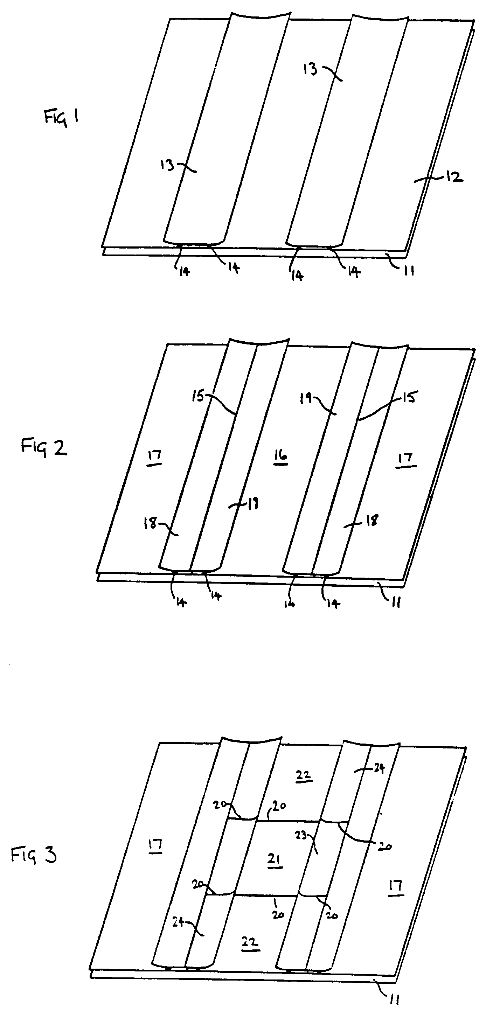

Referring to Figure 1, an adhesive-coated polyurethane film 11 is provided with a release paper 12. Two strips 13 of handling tab material are attached to the upper surface of release paper 12 by means of pairs of lines of adhesive 14.

Figure 2 shows the dressing of Figure 1 after a first die-cutting step. Two lines of cut 15 between the lines of adhesive 14, separate the release paper into a central region 16 and two end regions 17. Each end region 17 now has a handling tab 18, while the central region 16 has two handling tabs 19.

In an alternative method, the partially-formed dressing shown in Figure 2 may be formed by making the first die cut prior to applying the strips of handling tab material. In this case, of course, it is then necessary to apply four separate strips 18, 18, 19, 19. In a further variation, a narrow double sided pressure sensitive adhesive tape may be used instead of a line of holt melt adhesive. This method is preferred if either of the materials being bonded is temperature sensitive.

Figure 3 shows the dressing of Figure 2 after a second die-cutting step. Two lines of cut 20 have now separated the central region 16 into an inner portion 21 and two outer sections 22, each with respective pairs of handling tabs 23, 24. The inner portion 21 may now easily be removed, to expose a region of adhesive-coated polyurethane film 11, to which is then attached a pad of absorbent material such as calcium alginate fibre. The outer sections 22 and end regions 17 of the release paper act as a mask during this step to prevent particles of the pad material from contaminating portions of the adhesive which are required for attachment of the dressing to the patient.

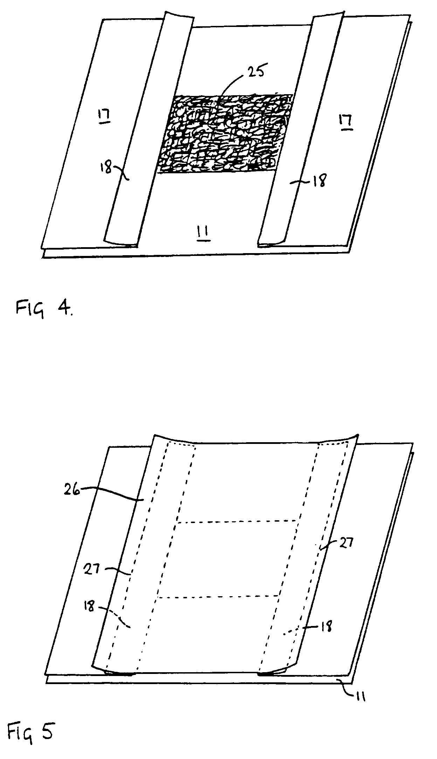

Figure 4 shows the dressing after attachment of the absorbent pad 25 and after removal of the outer sections 22 of the release paper. The regions of adhesive-coated film which are thereby exposed now serve to secure a protective film 26 (Figure 5) which extends over the full width of the dressing and beyond the outer edges 27 of the handling tabs 18. This means that the protective film may readily be grasped by the user of the dressing and removed. The exposed central region 16 of the dressing is then applied to the wound. For this operation, the dressing can conveniently be handled by its two end regions, which remain protected by their respective portions of release paper. These latter are then removed by grasping their respective tabs and peeling the release papers away from the film, which is then smoothed onto the patient's skin.

It will of course be understood that the present invention has been described above purely by way of example, and modifications of detail can be made within the scope of the invention. For example, the absorbent pad may be circular, rather than rectangular. In such a case, when manufactured by the preferred method of the present invention, the release lamella is cut to produce a circular inner portion.

## Claims

1. A method of forming a wound dressing comprising the steps of
i) providing an adhesive-coated plastics film having a lamella releasably attached to its adhesive surface,
ii) cutting the lamella into an inner and an outer portion, such that the outer portion surrounds the inner portion,
iii) removing the inner portion to expose a region of the adhesive-coated plastics film, and
iv) attaching a layer of absorbent material to said exposed region of film.

2. A method according to claim 1 wherein, after attachment of the absorbent layer, the outer portion of lamella is removed in whole or in part to expose a further region of adhesive-coated film, to which is then attached one or more protective films to prevent soiling of the absorbent layer prior to use.

3. A method according to claim 1 or claim 2 wherein, prior to removal of the inner portion of the lamella, a handling tab is adhesively secured to said inner portion.

4. A method according to claim 1 or claim 2 wherein the absorbent material is an alginate.

## Patentansprüche

1. Verfahren zur Herstellung eines Wundverbands umfassend die Schritte:
i) Bereitstellen eines mit Klebstoff beschichteten Kunststoffilms, an dessen Klebfläche ein abziehbares Blättchen befestigt ist,
ii) Zuschneiden des Blättchens in einen inneren und einen äußeren Teil, so daß der äußere Teil den inneren Teil umgibt,
iii) Entfernen des inneren Teils, um einen Bereich des mit Klebstoff beschichteten Kunststoffilms freizulegen, und
iv) Befestigen einer Schicht absorbierenden Materials an der genannten freigelegten Fläche des Films.

2. Verfahren nach Anspruch 1, wobei, nach Anbringung der absorbierenden Schicht, der äußere Teil des Blättchens zur Gänze oder teilweise entfernt wird um einen weiteren Bereich des mit Klebstoff beschichteten Films freizulegen, auf den dann ein oder mehrere Schutzfilme angebracht werden, um eine Verschmutzung der absorbierenden Schicht vor der Benutzung zu verhindern.

3. Verfahren nach Anspruch 1 oder 2, wobei, vor dem Entfernen des inneren Teils des Blättchens, ein Handhabungslasche haftend an dem inneren Teil befestigt ist.

4. Verfahren nach Anspruch 1 oder 2, wobei das absorbierende Material ein Alginat ist.

## Revendications

1. Un procédé pour former un pansement pour blessure comportant les étapes consistant à:
i) fournir un film plastique revêtu d'adhésif ayant une lamelle fixée de façon détachable à sa surface adhésive,
ii) découper le lamelle en une partie interne et une partie externe, de telle sorte que la partie e'terne entoure la partie interne,
iii) enlever la partie interne pour exposer une zone du film plastique revêtu d'adhésif, et
iv) fixer une couche de matériau absorbant à ladite zone exposée du film.

2. Un procédé selon la revendication 1, dans lequel, après fixation de la couche absorbante, la partie externe de la lamelle est enlevée dans sa totalité ou en partie pour exposer une zone supplémentaire du film revêtu d'adhésif, à laquelle est ensuite fixé un ou plusieurs films protecteurs pour empêcher de salir la couche absorbante avant utilisation.

3. Un procédé selon la revendication 1 ou la revendication 2, dans lequel, avant l'enlèvement de la partie interne de la lamelle, une patte de préhension est fixée, de façon adhésive, à ladite partie interne.

4. Un procédé selon la revendication 1 ou la revendication 2, dans lequel le matériau absorbant est un alginate.
